(19) Europäisches Patentamt
European Patent Office
Office européen des brevets

(11) EP 0 974 365 B1

(12) **FASCICULE DE BREVET EUROPEEN**

(45) Date de publication et mention de la délivrance du brevet:
**22.09.2004 Bulletin 2004/39**

(51) Int Cl.[7]: **A61K 47/32**, A61K 9/20

(21) Numéro de dépôt: **99401649.1**

(22) Date de dépôt: **01.07.1999**

(54) **Utilisation d'un polymère de type acrylique en tant qu'agent de désagrégation, procédé pour faire des comprimés et comprimés en résultant**

Verwendung eines Acrylsäure-Typ Polymeres als Desintegrationsmittel, Vefahren zur Herstellung von Tabletten und hergestellte Tabletten

Use of an acrylic-type polymer as desintregrating agent, process for making tablets and resulting tablets

(84) Etats contractants désignés:
**AT BE CH CY DE DK ES FI FR GB GR IE IT LI LU MC NL PT SE**
Etats d'extension désignés:
**SI**

(30) Priorité: **20.07.1998 FR 9809221**

(43) Date de publication de la demande:
**26.01.2000 Bulletin 2000/04**

(73) Titulaire: **PERMATEC TECHNOLOGIE AG**
**6301 Zug (CH)**

(72) Inventeurs:
- **Rault, Isabelle**
  **68100 Mulhouse (FR)**
- **Pionnier, Etienne**
  **68100 Mulhouse (FR)**

(74) Mandataire: **Peaucelle, Chantal et al**
**Cabinet Armengaud Aine**
**3, avenue Bugeaud**
**75116 Paris (FR)**

(56) Documents cités:
- **MCGINITY, JAMES W. ET AL: "Controlled-release theophylline tablet formulations containing acrylic resins. I. Dissolution properties of tablets" DRUG DEV. IND. PHARM. (1983), 9(1-2), 57-68 CODEN: DDIPD8;ISSN: 0363-9045, XP002100601**
- **CAMERON, CLAUD G. ET AL: "Controlled-release theophylline tablet formulations containing acrylic resins. II. Combination resin formulations" DRUG DEV. IND. PHARM. (1987), 13(8), 1409-27 CODEN: DDIPD8;ISSN: 0363-9045, XP002100602**
- **GIDWANI, RAM ET AL: "Spray-dried enteric solid dispersion as a novel oral delivery system for pentapeptide analog of thymopentin" DRUG DEV. IND. PHARM. (1992), 18(4), 385-94 CODEN: DDIPD8;ISSN: 0363-9045, XP002100603**
- **ERDOS, S.: "Spontaneous disintegration" PHARM. IND. (1986), 48(5), 503-7 CODEN: PHINAN;ISSN: 0031-711X, XP002100604**
- **OFOEFULE, SABINUS IFEANYI: "Effect of polyethylene glycol 4000 (PEG 4000) solution on the in vitro release profile of nifedipine from polymer matrixes" BIOL. PHARM. BULL. (1997), 20(5), 574-576 CODEN: BPBLEO;ISSN: 0918-6158, XP000656147**
- **KRISTOFFERSSON, EEVA ET AL: "Theophylline tablet formulations. Importance of the composition of tablets, the compression force used in tableting and the in vitro stirrin speed on the release of theophylline from two-layer tablets" ACTA PHARM. FENN. (1978), 87(1), 9-19 CODEN: APHFDO;ISSN: 0356-3456, XP002100606**
- **DATABASE WPI Section Ch, Week 8042 Derwent Publications Ltd., London, GB; Class A96, AN 80-73552C XP002100608 & DD 143 213 A (DITTGEN M), 13 août 1980 (1980-08-13)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 974 365 B1

Printed by Jouve, 75001 PARIS (FR)

(Cont. page suivante)

- **LEHMANN, KLAUS: "Formulation of controlled release tablets with acrylic resins" ACTA PHARM. FENN. (1984), 93(2), 55-74 CODEN: APHFDO;ISSN: 0356-3456, XP002100607**


Remarques:

Le dossier contient des informations techniques présentées postérieurement au dépôt de la demande et ne figurant pas dans le présent fascicule.

## Description

La présente demande est, de manière générale, relative à l'utilisation d'un polymère de type acrylique en tant qu'agent de délitement ou de désagrégation. Elle est en particulier relative à l'utilisation d'un copolymère de type acrylique en tant qu'agent, ou co-agent de désagrégation dans une forme galénique de type comprimé, ainsi qu'à un procédé de fabrication de formes galéniques de type comprimés mettant en oeuvre un tel copolymère en tant qu'agent, ou co-agent de désagrégation.

Les agents les plus généralement utilisés pour la fabrication de formes galéniques destinées à se désagréger correspondent à des agents dits désintégrants du type carboxyméthyl cellulose sodique, ou à des agents dits de gonflement du type crospovidone ou amidon modifié .

Si certains de ces agents permettent l'obtention de comprimés présentant une vitesse de désagrégation adaptée aux conditions de leur utilisation, tout en présentant des caractéristiques pharmacotechniques acceptables (notamment, dureté, friabilité et stabilité), ce n'est qu'au prix de procédés longs et compliqués. En effet, l'utilisation de ces agents à titre d'agent de désagrégation implique par exemple la réalisation de sous-structures particulières telles que microparticules ou microgranules avant mise en forme galénique finale du comprimé, ou bien la mise en oeuvre de réactions de type effervescence, réactions qui elles-mêmes contraignent à fabriquer les comprimés dans un milieu à humidité réduite.

Le document DRUG DEV. IND. PHARM. (1983), 9(1-2), 57-68 divulgue des comprimés obtenus par compression directe de copolymères méthacryliques Eudragit RSPM$^R$ , RLPM$^R$, S100$^R$ et L100$^R$. Le document PHARM. IND. (1986), 48(5), 503-7 divulgue la désintégration spontanée de comprimés comprenant de l'Eudragit L$^R$.

L'invention objet de la présente demande propose une nouvelle utilisation d'un polymère de type acrylique et un nouveau procédé qui visent à pallier les inconvénients attachés aux techniques de l'art antérieur. La nouvelle utilisation et le nouveau procédé selon l'invention font de plus preuve de performances particulièrement appréciables pour l'obtention de comprimés qui présentent, dans des conditions d'usage ou équivalentes à cet usage, des propriétés de désagrégation appropriées, tout en présentant, avant usage, et en particulier lors du stockage, de très bonnes caractéristiques pharmacotechniques (notamment, dureté, friabilité, stabilité).

Les polymères de type acrylique sont, quant à eux, généralement utilisés dans l'art antérieur pour la fabrication de comprimés à libération prolongée, et/ou pour la réalisation d'enrobages entériques. Parmi les différents polymères de type acrylique connus de l'homme du métier, la pharmacopée US National Formulary (USP/NF) distingue notamment des copolymères d'acide méthacrylique de type A, B ou C, et des copolymères de méthacrylate d'ammonium de type A ou B. Il est en particulier connu que les copolymères d'acide méthacrylique de type A ou B, et les copolymères de méthacrylate d'ammonium de type A ou B peuvent être utilisés dans la réalisation de matrices retards (dépendantes du pH pour les premiers copolymères ; indépendantes du pH pour les seconds). Les copolymères d'acide méthacrylique de type C sont quant à eux connus pour pouvoir être utilisés dans la réalisation de différents enrobages : enrobages entériques du fait de leurs propriétés de gastro-résistance, et de leur solubilité en milieu intestinal à pH 5,5-7,5 ; enrobages isolants destinés à la protection des principes actifs sous environnement de type tropical ; ou bien encore enrobages destinés à masquer le goût ou l'odeur.

Les inventeurs ont mis en évidence que certains polymères de type acrylique, à savoir les copolymères d'acide méthacrylique de type C selon l'USP/NF, sont, de manière inattendue, capables d'améliorer très nettement la vitesse de désagrégation d'un comprimé, tout en permettant l'obtention d'un comprimé présentant de très bonnes caractéristiques pharmacotechniques, et notamment une très bonne cohésion. L'utilisation d'un copolymère d'acide méthacrylique de type C selon l'invention présente notamment l'avantage de permettre l'obtention de comprimés à désagrégation rapide, et en particulier à désagrégation de type immédiat, qui présentent, avant usage, de très bonnes caractéristiques pharmacotechniques (notamment, de très bonnes caractéristiques, de dureté et de friabilité).

Ces très bonnes caractéristiques pharmacotechniques racilitent le conditionnement et le stockage des comprimés ainsi produits : les comprimés produits à l'aide de l'utilisation ou du procédé selon l'invention ne nécessitent pas un conditionnement spécifiquement adapté à la protection de la structure de ces comprimés comme des conditionnements avec un film pelable de façon à éviter l'extraction du comprimé hors de l'alvéole par pression du doigt.

Ces très bonnes caractéristiques pharmacotechniques sont également à l'origine des très bonnes caractéristiques de conservation dans le temps desdits comprimés (stabilité).

Ces polymères de type acrylique particuliers selon l'invention présentent également, outre la surprenante efficacité de désagrégation qu'ils procurent, l'avantage notable de la facilité d'utilisation pour la fabrication de comprimés.

Ils ne nécessitent en effet pas, en eux-mêmes, la mise en oeuvre de technologies particulières pour la réalisation de comprimés cohésifs à désagrégation rapide : toute technologie connue de l'homme du métier peut être mise en oeuvre, si tant est que les autres éléments de la forme galénique sont choisis de manière à le permettre. Ils permettent en particulier la réalisation de comprimés cohésifs à désagrégation rapide par simple compression directe, sans nécessiter la mise en oeuvre de technologies astreignantes telle que la granulation humide.

La présente demande a donc pour objet, de manière générale, l'utilisation d'au moins un copolymère d'acide méthacrylique de type C selon l'USP/NF à titre d'agent, ou co-agent, permettant d'améliorer la vitesse de désagrégation d'un comprimé, tout en permettant l'obtention d'un comprimé de bonne cohésion, notamment par simple compression directe. Elle vise notamment, l'utilisation d'au moins un copolymère d'acide méthacrylique de type C selon l'USP/NFà titre d'agent, ou co-agent, permettant, ou participant au désagrégation d'un comprimé, ainsi qu'un procédé de fabrication de comprimés mettant en oeuvre au moins un tel copolymère en tant qu'agent, ou co-agent, de désagrégation dans un comprimé.

L'invention de la présente demande propose un procédé de fabrication d'un comprimé, caractérisé en ce qu'il comprend la mise en oeuvre d'un copolymère d'acide méthacrylique de type C selon la pharmacopée US National Formulary USP/NF, en tant qu'agent ou co-agent de désagrégation dudit comprimé, et en particulier en tant qu'agent ou co-agent de désagrégation de type immédiat dudit comprimé, caractérisé en ce qu'il comprend en outre au moins une étape de mise en forme galénique par simple compression directe, sans nécessiter la mise en oeuvre d'une étape de granulation humide

L'invention concerne également le comprimé susceptible d'être obtenu par le procédé.

Elle vise en particulier l'utilisation d'au moins un copolymère d'acide méthacrylique de type C selon l'USP/NF à titre d'agent, ou co-agent, permettant, ou participant au désagrégation rapide, et notamment au désagrégation rapide de type immédiat, d'un comprimé ainsi qu'un procédé de fabrication de comprimés à désagrégation rapide, et notamment à désagrégation immédiat, mettant en oeuvre un tel copolymère à titre d'agent, ou co-agent de désagrégation.

L'effet de désagrégation de comprimé observé selon l'invention ne correspond pas à une simple érosion de type mécanique, mais plutôt à un effet de type gonflement après hydratation appropriée du comprimé. Par "agent de désagrégation dans un comprimé", nous entendons, dans la présente demande, un agent permettant une amélioration de la vitesse de désagrégation observée pour ce comprimé en l'absence de cet agent.

Cet effet d'amélioration de la vitesse de désagrégation du comprimé peut naturellement être optimisé en choisissant les autres caractéristiques de comprimé (telles que nature et charge des autres composants du comprimé, masse, format, dureté de ce comprimé) de manière à ce qu'elles ne s'opposent pas, voire à ce qu'elles favorisent ce phénomène de désagrégation.

Par "agent de désagrégation rapide dans un comprimé", nous entendons ainsi, dans la présente demande, un agent permettant une amélioration significative de la vitesse de désagrégation de ce comprimé, telle que ci-dessus présentée.

Le terme "significative" peut être apprécié à l'aide de tout outil statistique connu de l'homme du métier. Des conditions appropriées pour observer cette amélioration significative comprennent celles qui consistent à placer ledit comprimé dans des conditions de milieu, et notamment de composition, de pH et de température adaptées au désagrégation du comprimé considéré.

Par "agent de désagrégation de type immédiat dans un comprimé", nous entendons un agent permettant le désagrégation dudit comprimé dans un temps inférieur ou égal à 25s environ, préférentiellement inférieur ou égal à 20s environ, encore plus préférentiellement inférieur ou égal à 10s environ, lorsque ce comprimé est testé dans des conditions appropriées à son désagrégation, et lorsque les autres composants du comprimé et sa structure (masse, format, dureté) sont choisis de manière à ne pas s'opposer, voire à favoriser, ce phénomène de désagrégation. Des conditions appropriées pour tester le désagrégation d'un comprimé comprennent des conditions mimant celles sous lesquelles ledit comprimé est destiné à se déliter. Par exemple dans le cas d'un comprimé destiné à se déliter dans les conditions physiologiques d'une cavité buccale, de telles conditions appropriées comprennent le fait de tester ledit comprimé sur un appareil de type Erweka ZT3® en milieu salivaire à 33°C et pH 6,0.

Le terme "agent", utilisé dans la présente demande, couvre également une situation de co-agent. Ainsi, l'utilisation selon l'invention comprend avantageusement la mise en oeuvre d'un copolymère d'acide méthacrylique de type C selon l'USP/NF en tant qu'agent de désagrégation de type immédiat selon l'invention, couplée à la mise en oeuvre d'un ou plusieurs agent(s) de désagrégation connu(s) tel que la crospovidone (par exemple, celle commercialisée sous le nom de marque Kollidon-CL® par BASF Aktiengesellschaft, Ludwigshafen, Allemagne).

Le(s) copolymère(s) d'acide méthacrylique de type C utilisé(s) en tant qu'agent(s), ou co-agent(s) de désagrégation selon l'invention peut (peuvent) être notamment utilisé(s) pour la fabrication de tout comprimé nécessitant une amélioration de la vitesse de désagrégation, et notamment une vitesse de désagrégation élevée. C'est en particulier le cas de comprimés adaptés, ou destinés, à une application pharmaceutique, vétérinaire, ou hygiénique.

Peuvent être notamment cités les comprimés pharmaceutiques, vétérinaires ou hygiéniques destinés à une administration par voie orale pour un désagrégation dans la cavité buccale, ceux destinés à une administration par voie orale pour un désagrégation différé, par exemple, différé au niveau des intestins, ou bien encore ceux destinés à une administration par voie vaginale.

La présente demande vise en particulier l'utilisation d'un copolymère d'acide méthacrylique de type C selon l'USP/NF dans un comprimé destiné à se déliter rapidement notamment (de type immédiat) dans la cavité buccale. Un tel copolymère ainsi utilisé, ou mis en oeuvre, ou en tant qu'agent (ou co-agent) de désagrégation permet en effet

l'obtention de comprimés capables (après hydratation par la salive) de se déliter dans la cavité buccale dans des temps très courts (de manière particulièrement préférentielle, des temps inférieurs à 10s sont obtenus). La suspension ainsi créée bénéficie d'une grande surface d'échange, et les principes actifs sont libérés de manière particulièrement rapide. Outre l'intérêt de cette forme galénique sur le plan de la biodisponibilité, elle présente d'autres avantages dont un des principaux est sans nul doute sa facilité d'emploi. Le patient n'a en effet pas besoin d'eau pour prendre son traitement, d'où une meilleure observance. Pour les jeunes enfants, ou les personnes âgées, cette forme ne nécessite par ailleurs pas de mastication, ni d'effort de déglutition.

Les comprimés à désagrégation buccal peuvent toutefois présenter l'inconvénient d'un goût et/ou une odeur désagréable. Pour diminuer, voire neutraliser l'odeur et/ou le goût déplaisant de la forme galénique, il peut utilisé l'ajout d'édulcorants et/ou d'arômes dans la formulation, et/ou être utilisé l'enrobage de ceux des composants qui présentent un goût et/ou une odeur désagréable (par exemple, enrobage de principe actif). De manière remarquable, de tels composants modifiés par enrobage peuvent être compris dans l'utilisation selon l'invention sans modifier de façon notoire sa vitesse de désagrégation.

Les proportions dans lesquelles ledit (ou chacun desdits) copolymère(s) d'acide méthacrylique de type C doit (doivent) être utilisé(s) selon l'invention peuvent être aisément testées par essai et erreur à l'aide de techniques connues de l'homme du métier, selon la formulation complète de comprimé choisie, et selon l'effet recherché. A titre indicatif, ces proportions sont généralement comprises de 5 à 50% environ de la masse totale du comprimé.

Le (ou les) copolymère(s) d'acide méthacrylique de type C mis en oeuvre, ou utilisé(s) en tant qu'agent(s), ou co-agent(s) de désagrégation selon l'invention n'entraîne(nt) aucune restriction quant à la nature possible des autres éléments de la forme galénique. Ainsi, il(s) peut (peuvent) être associé(s) à toute substance ou tout excipient approprié au type d'application auquel le comprimé est destiné.

Selon une disposition de l'invention, ladite utilisation met à profit le fait que les copolymères d'acide méthacrylique de type C sont à la fois gastro-résistants, et solubles en milieu intestinal, pour les associer à un ou plusieurs copolymère(s) de méthacrylate d'ammonium de type A et/ou B selon l'USP/NF (matrice retard indépendante du pH), de manière à favoriser la libération du principe actif dans l'intestin. Le procédé et l'utilisation selon l'invention permettent alors d'obtenir un comprimé destiné à une administration par voie orale, qui ne se délite qu'au niveau des intestins, et cela dans des temps très courts, voire des temps de type immédiat si nécessaire.

Pour une application par voie vaginale, l'utilisation selon l'invention peut en outre comprendre la mise en oeuvre d'un composé bioadhésif.

De manière tout-à-fait générale, l'utilisation d'au moins un copolymère d'acide méthacrylique de type C selon l'invention comprend en outre l'utilisation d'un principe actif ou d'un placebo, l'utilisation d'un diluant (tel que le dextrose), d'un agent favorisant la dureté (tel que le sorbitol), d'un lubrifiant (tel que le stéarate de magnésium ou l'huile d'arachide), et éventuellement d'un liant (tel que l'amidon de maïs).

De manière plus particulière, l'utilisation selon l'invention peut également comprendre en outre l'utilisation d'excipients ou de substances parmi ceux jouant les rôles :

- d'agents de désagrégation ou de désagrégation comme par exemple la crospovidone (commercialisée sous le nom de marque Kollidon-CL® par exemple), la croscarmellose sodique, le carboxyméthylamidon sodique, l'hydroxypropylcellulose partiellement substituée, le glycolate d'amidon sodique, dans une proportion pouvant varier de 1 % à 50 %,
- des agents gonflants solubles ou peu solubles,
- de diluants comme le lactose,
- de liants comme l'amidon de maïs,
- de lubrifiants comme le stéarate de magnésium,
- d'agents d'écoulement comme la silice colloïdale,
- des agents de solubilisation,
- des aromatisants,
- des édulcorants,
- des plastifiants,
- des antioxydants,
- des agents de pelliculage, d'enrobage,
- des agents entrant dans la composition de la solution de polissage et brillantage,
- les agents assurant une protection thermique du principe actif comme les dérivés de saccharose,
- des excipients ou substances assurant une bioadhésion comme les

dérivés de l'acide acrylique, le copolymère de méthylvinyléther et d'anhydride maléique, la gomme guar, la gomme xanthane, le caroube, les carraghénates, la pectine, une protéine biologique ou synthétique seule ou en association avec d'autres protéines d'origine biologique ou synthétique, les cyclodextrines, les hydroxypropylbé-tacyclodextrines,

les bétacyclodextrines et leurs dérivés.

L'utilisation selon l'invention permet d'associer au comprimé tout principe actif désiré. Peuvent notamment être cités les antihistaminiques, les anticholergéniques, les éléments minéraux, les allergènes, les anesthésiants de surface, locaux ou généraux, les antipyrétiques, les antalgiques non opiacés, les antalgiques opiacés, les antispasmodiques anticholinergiques et non anticholinergiques, les anti-inflammatoires non stéroïdiens tels que l'acide tiaprofénique, l'indométhacine, le diclofénac, l'ibuprofène, le kétoprofène, le naproxène, le piroxicam, les anti-inflammatoires stéroïdiens comme la béthamétasone, la prédnisolone, les cytotoxiques, les agents antihormonaux, les antianémiques, les antiémétiques, les antiasthéniques, les antihypertenseurs et parmi eux les béta-bloquants comme le propranolol, l'aténolol, le métoprolol, les inhibiteurs de l'enzyme de conversion comme le captopril, l'énalapril, les antagonistes de l'angiotensine II, les inhibiteurs calciques tels que la nifédipine et le diltiazem, les antihypertenseurs d'action centrale, les vasodilatateurs, les hypolipémiants, les antidiabétiques oraux, les anticoagulants, les antiaggrégants plaquettaires, les inhibiteurs calciques, les dérivés nitrés utilisés dans le traitement de l'insuffisance coronarienne, les antiangoreux non nitrés, les diurétiques, les dérivés de la digitaline et apparentés, les antiarythmiques, les antihypotenseurs et analeptiques circulatoires, les vasodilatateurs, les anti-ischémiques, les vasculoprotecteurs et les veinotoniques, les hormones, les antiherpétiques, les antiphotosensibilisants, les antiulcéreux comme la ranitidine, la cimétidine, les antiacides, les laxatifs, les antidiarrhéiques, les antifongiques, les cholélitholytiques, les interférons, les enzymes, les antispasmodiques, les antibactériens, les antiseptiques, les antiherpétiques, les utérorelaxants, les ocytociques, les oestrogènes, les progestatifs, les ostroprogestatifs, les principes actifs indiqués dans la lactation comme la bromocriptine, les principes actifs indiqués dans le traitement de la stérilité, les antigonadotropes, les anticoagulants, les thrombolytiques, les antifibrinolytiques, les vitamines, les hémostatiques, les cyclosporines, des agents alkylants, des antibiotiques, les antiviraux, les antiparasitaires, les vaccins, les produits de diagnostic, les principes actifs indiqués dans le traitement de l'obésité, les oréxigènes, les principes actifs indiqués dans le traitement des corrections des anomalies métaboliques, les principes actifs indiqués dans la nutrition orale et entérale, les agents anticonvulsifs, les antiparkinsoniens, les antimyasthéniques, les principes actifs indiqués dans le traitement de la maladie d'Alzheimer, les antimigraineux, les neuroleptiques, les anxiolytiques, les hypnotiques, les sédatifs, les antidépresseurs, les normothymiques, les psychostimulants, les principes actifs indiqués dans le traitement des états de dépendance en alcoologie, en désintoxication tabagique, en désintoxication des opiacées, les antiglaucomateux, les mydriatiques, les brochodilatateurs, les antiasthmatiques, les antitussifs, les fluidifiants bronchiques, les révulsifs (topiques), les principes actifs indiqués dans le traitement des ostéopathies, les principes actifs indiqués dans le traitement dans l'accès aigu de goutte, les principes actifs indiqués dans le traitement hypo-uricémiant, les principes actifs indiqués dans le traitement des algodystrophies, les myorelaxants, les principes actifs indiqués dans le traitement de l'arthrose, les correcteurs des hyposialies, les principes actifs indiqués dans le traitement de la lithiase urinaire, les principes actifs indiqués dans le traitement de l'insuffisance rénale, les principes actifs indiqués dans le traitement de l'énurésie, les principes actifs indiqués dans le traitement de l'éjaculation rétrograde, les principes actifs indiqués dans le traitement de l'impuissance.

Le(s) copolymère(s) d'acide méthacrylique de type C selon l'USP/NF utilisé(s) en tant qu'agent de désagrégation selon l'invention peut (peuvent) être incorporé(s) en mélange dans la masse du comprimé, ou bien ne faire partie que de certaines sous-structures de comprimé, par exemple être incorporé(s) dans des micro- ou nano-particules (ou micro- ou nano-capsules) incluses dans un comprimé, et/ou être incorporé(s) dans une couche d'un comprimé multicouche, en particulier dans une couche destinée à un désagrégation rapide. Ce comprimé, destiné à un désagrégation rapide, qu'il s'agisse d'un monocouche, d'un particulaire, ou d'un multicouche, ou d'une combinaison de ces dispositions, se présente avantageusement sous la forme d'un comprimé bioadhésif (par exemple, pour un désagrégation rapide par voie vaginale), et/ou d'un comprimé pour la libération rapide mais différée dans le temps du (ou des) principe(s) actif(s) (par exemple, désagrégation rapide au niveau des intestins après administration par voie orale), ou d'un comprimé pour la libération rapide et immédiate du (ou des) principe(s) actif(s) (par exemple, désagrégation rapide dans la bouche).

De manière avantageuse, l'utilisation selon l'invention comprend une utilisation de ladite masse de comprimé, ou, le cas échéant, de ladite sous-structure de comprimé sous une forme essentiellement pulvérulente avant mise en forme galénique.

L'utilisation d'un copolymère d'acide méthacrylique de type C en tant qu'agent de désagrégation d'un comprimé selon l'invention permet l'obtention dudit comprimé (ou, le cas échéant, l'obtention de ladite sous-structure de comprimé dans laquelle ledit copolymère est incorporé) à l'aide de toute technique connue de l'homme du métier, telle que granulation humide, granulation sèche ou compactage, extrusion, ainsi que, de manière avantageuse, telle que compression directe. Une restriction à un ou plusieurs types de technique peut être observée selon la nature et/ou la proportion des autres composants utilisés dans la fabrication dudit comprimé, et/ou selon la structure que l'on souhaite donner à ce comprimé. Une telle restriction n'est toutefois pas le fait dudit copolymère d'acide méthacrylique de type C utilisé. De manière préférentielle, l'utilisation selon l'invention comprend une mise en forme galénique dudit comprimé, ou le cas échéant, de ladite sous-structure de comprimé, par simple compression directe.

De manière remarquable, l'utilisation selon l'invention permet l'obtention de comprimés qui présentent de très bonnes caractéristiques pharmacotechniques, et notamment qui présentent, après mise en forme galénique, une dureté comprise entre 1,96133 N et 98,0665 N (0,2 et 10 Kp) environ et une friabilité comprise entre 0 et 25% environ.

L'utilisation selon l'invention est adaptée à la réalisation de comprimés de toute masse et tout format, sans limitation. Classiquement, pour des applications médicales, vétérinaires ou hygièniques, des masses de comprimé allant de 50 à 200 mg sont réalisées et des formats de types D6R4, D6 plat, D7R5, D9R7, D9 plat, D10 plat sont utilisés.

De manière particulièrement remarquable, l'utilisation selon l'invention permet l'obtention de comprimés qui, tout en présentant de très bonnes caractéristiques pharmacotechniques, sont capables de se déliter dans un temps inférieur à 25s environ, préférentiellement dans un temps inférieur ou égal à 20s environ, encore plus préférentiellement inférieur ou égal à 10s environ, lorsqu'ils sont placés dans des conditions appropriées à leur désagrégation. La détermination de conditions appropriées est connue de l'homme du métier, et des exemples en sont donnés dans la présente demande.

Des exemples de copolymères d'acide méthacrylique de type C selon USP/NF qui peuvent être utilisés selon l'invention comprennent ceux commercialisés par la société Rôhm GmbH (Darmstadt, Allemagne), sous le nom d'Eudragit L100-55® (forme pulvérulente), ou d'Eudragit L30D-55® (dispersion aqueuse). L'Eudragit L100-55® répond à la formule suivante :

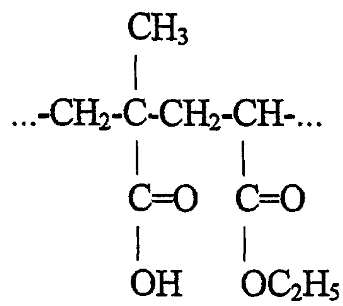

La présente invention a également pour objet un procédé de fabrication de comprimés, notamment de comprimés à désagrégation rapide, et en particulier à désagrégation rapide de type immédiat, ledit procédé mettant en oeuvre au moins un copolymère d'acide méthacrylique de type C, selon l'USP/NF en tant qu'agent, ou co-agent de désagrégation, et en particulier mettant en oeuvre au moins un copolymère d'acide méthacrylique de type C tel qu'utilisé selon l'invention. De manière préférentielle et avantageux, le procédé selon l'invention comprend en outre la mise en forme galénique des comprimés par simple compression directe.

Les exemples qui suivent sont donnés à titre illustratif.

Dans ces exemples, différents paramètres de pharmacotechnie sont mesurés à l'aide des techniques classiques. Parmi ces paramètres, peuvent être notamment cités la dureté, la friabilité, ainsi que la stabilité du comprimé obtenu selon l'utilisation et/ou le procédé de l'invention.

Parmi les moyens disponibles à l'homme du métier pour la mesure de tels paramètres, peuvent être cités :

- un appareil de type Schleuninger 2E/205 pour la mesure de la dureté,
- un friabilimètre à tambour de type tambour à ailette (Roche) pour la mesure de la friabilité, un protocole de mesure de friabilité adapté comprenant le fait de placer un échantillon représentatif de comprimés (par exemple, 10 comprimés) dans un tel tambour en rotation en sens horaire pendant 10 min à 25 tr/min, de mesurer la masse moyenne d'un comprimé avant ce traitement ($m_{to}$), et après dépoussiérage suite à ce traitement ($m_{10min}$), et de calculer le % de friabilité comme suit :

$$\% \text{ de friabilité} = \frac{m_{t10min}}{(m_{to} - m_{t10\,min})} \times 100$$

- un appareil à désagrégation de comprimés de type Erweka ZT3 suivant un protocole de mesure adapté comprenant le fait de placer 1 comprimé dans chacun des 6 tubes en verre du panier, puis d'ajouter le disque. Le panier est ensuite suspendu et maintenu au dessus du bécher rempli de milieu salivaire. La mesure du temps de désagrégation s'effectue à partir de la descente du panier dans le bécher jusqu'à désagrégation complète des comprimés. Composition du milieu salivaire utilisé (pH = 6) : KCl : 1,20 g/l ; $MgCl_2$, $6H_20$ : 0,05 g/l ; $CaCl_2$, $6H_20$: 0,15 g/l ;

KSCN : 0,10 g/l.

- des enceintes climatiques pour la mesure de la stabilité, un protocole de mesure adapté comprenant le fait de suivre des paramètres pharmacotechniques telles que dureté et friabilité au cours du temps (par exemple tous les trois mois) sur un échantillon représentatif de comprimés placés sous différentes conditions climatiques (par exemple à 25°C et à 45°C), et/ou le fait de suivre la stabilité d'un composé (par exemple un principe actif) initialement inclus dans de tels comprimés.

Exemple 1 :

Des comprimés sont réalisés par compression directe en suivant la formule "Eudragit" indiquée dans le tableau 1 ci-dessus. Des comprimés répondant à la formule témoin sont réalisés dans des conditions équivalentes. Ces formules sont choisies de manière appropriée pour permettre, après mise en forme galénique, et lors de leur usage, un désagrégation rapide dans la cavité buccale.

Les comprimés présentent un format plat, chanfreiné, et un diamètre égal à 8 mm. Ils sont terminés à 205 mg chacun.

Tableau 1 :

| Excipients | Formule Eudragit (% en masse) | Formule témoin (% en masse) |
|---|---|---|
| Eudragit L100-55® (Rhôm, SPCI) | 30,00 | - |
| Kollidon-CL® (BASF) | 15,00 | 15,00 |
| Dextrose (Roquette) | 51,70 | 81,70 |
| Vanille de synthèse (SBI) | 3,00 | 3,00 |
| Huile d'arachide | 0,30 | 0,30 |

Les paramètres de pharmacotechnie, et notamment de dureté moyenne et de temps de désagrégation moyen de ces deux groupes de comprimés sont ensuite mesurés. Les résultats obtenus sont illustrés par le tableau 2 cidessous.

Tableau 2 :

| Formule | Dureté N (Kp) | Temps de désagrégation en seconde (*) |
|---|---|---|
| Formule Eudragit | 15,69-21,57 (1,6-2,2)<br>1,6 -2,2 | 7 -13 |
| Formule Témoin | 11,76 - 14,70 (1,2-1,5)<br>1,2 - 1,5<br>34,32 (3,5)<br>3,5 | 20-25<br>25-30 |

* : temps de désagrégation testé sur appareil Erweka ZT3 en milieu salivaire à 33 °C à pH 6,0.

Les résultats pharmacotechniques indiquent que l'Eudragit L100-55®, qui est un copolymère d'acide méthacrylique de type C selon l'USP/NF, améliore significativement le temps de désagrégation d'un comprimé, pour atteindre des temps de désagrégation de type immédiat (inférieurs à 20s) environ. L'utilisation d'Eudragit L100-55® a permis de diminuer le temps de désagrégation du comprimé de 50 % en moyenne environ, tout en obtenant des comprimés de bonne cohésion. La friabilité des comprimés formule "Eudragit" est de 8,8 %.

Exemple 2 :

On peut également utiliser la formule "Eudragit" suivante (*cf.* tableau ci-dessous) pour obtenir, par compression directe, des comprimés placebo avec un temps de désagrégation de 7s (n=6), tel que mesuré en milieu salivaire à 33°C et pH 6,0 sur un appareil Erweka ZT3. Dans ce cas, on a remplacé l'huile d'arachide par le stéarate de magnésium et on a ajouté du sorbitol. Ces deux agents améliorent respectivement l'écoulement et la compressibilité du mélange de poudres. On peut travailler à des duretés plus faibles tout en obtenant un comprimé peu friable.

On réalise des comprimés par compression directe sur une machine à comprimer de type alternative Korsch EKO. On travaille sur 1,1 kg de mélange final de poudres, soit 10 000 comprimés terminés à 110 mg. On pèse 330 g

d'Eudragit L100-55® ; 165 g de Kollidon-CL® ; 535,7 g de dextrose ; 33 g de sorbitol et 33 g de vanille de synthèse (Givaudan-Roure). On mélange au turbula pendant 5 minutes ; on ajoute au mélange obtenu 3,3 g de stéarate de magnésium (magnésique). On mélange 30 secondes au turbula ; on passe le mélange en compression : le format des comprimés est D7R5 (diamètre de 7 mm et rayon de 5 mm) avec une épaisseur de 4 mm. Comme ci-dessous illustré, les comprimés obtenus ont une dureté de 3,92 N (0,4 Kp) une friabilité de 2,7 %, et un temps de désagrégation en milieu salivaire (appareil Erweka ZT3) à 33°C et pH 6,0 de 7 secondes.

Tableau 3 :

| Excipients | Formule Eudragit (% en masse) | Formule témoin (% en masse) |
|---|---|---|
| Eudragit L100-55® (Rhöm, SPCI) | 30,00 | - |
| Kollidon CL® (BASF) | 15,00 | 15,00 |
| Dextrose (Roférose G) (Roquette) | 48,75 | 78,75 |
| Sorbitol P 100 T (Roquette) | 3,00 | 3,00 |
| Vanille de synthèse (SBI) | 3,00 | 3,00 |
| Stéarate de magnésium | 0,25 | 0,25 |

Les paramètres pharmacotechniques des comprimés sont mesurés à l'aide des techniques classiques.

Les résultats de dureté et de temps de désagrégation de ces comprimés sont reportés dans le tableau 4 ci-dessous.

Tableau 4 :

| Formule | Dureté N (Kp) | Temps de désagrégation en seconde (*) | Friabilité en % |
|---|---|---|---|
| Formule Eudragit | 3,92 (0,4) | 7 | 2,7 |
| Formule Témoin | 10,78 (1,1) | 13 | 0,3 |

* : temps de désagrégation testé sur appareil Erweka ZT3 en milieu salivaire à 33 °C à pH 6,0.

L'utilisation de l'Eudragit L100-55 a permis de diminuer de près de la moitié le temps de désagrégation. Toutefois, il apparaît très clairement que l'Eudragit facilite également la compressibilité car il est impossible de travailler la formule témoin avec des forces de compression faibles (dureté du comprimé inférieure à 9,8 N (1 Kp). Cette dernière formule ne peut pas faire l'objet d'un développement industriel car elle ne remplit pas les caractéristiques galéniques nécessaires.

<u>Exemple 3</u> :

La formule utilisée en exemple 2 est réalisée de manière à obtenir des comprimés de 200 mg. Le format des comprimés est D9R7 (diamètre de 9 mm et rayon de courbure de 7 mm) avec une épaisseur de 5 mm.

Les paramètres pharmacotechniques des comprimés sont mesurés à l'aide des techniques classiques.

Les résultats de dureté et de temps de désagrégation de ces comprimés sont reportés dans le tableau 5 ci-dessous.

Tableau 5 :

| Formule | Dureté N (Kp) | Temps de désagrégation (s) | Friabilité en % |
|---|---|---|---|
| Formule Eudragit | 27,45 (2,8) | 16 | 0,9 |
| Formule témoin | 23,53 (2,4) | 23 | 0,5 |

Dans ce cas de figure, la formule Témoin ne peut pas être comprimée à une dureté inférieure à 19,61 N (2 Kp) ; les comprimés réalisés avec la formule Eudragit ont donc été fabriqués à une dureté comparable. On obtient, grâce à l'utilisation de l'Eudragit, une diminution du temps de désagrégation de l'ordre de 30 %.

<u>Exemple 4</u> :

L'utilisation d'un copolymère d'acide méthacrylique de type C (USP/NF) en tant qu'agent, ou co-agent, de

désagrégation selon l'invention permet de moduler la vitesse de désagrégation de comprimés. Cette modulation peut notamment être réalisée en jouant sur la quantité de copolymère d'acide méthacrylique de type C ajoutée.

Est ici présentée un exemple de modulation selon l'invention sur des comprimés chargés en Lopéramide en tant que principe actif.

Des comprimés sont réalisés en suivant la formule "Eudragit" indiquée dans le tableau 5 ci-dessus, par mélange des différentes poudres de la formule, puis mise en forme galénique par compression directe. Des comprimés témoin, sans copolymère d'acide méthacrylique de type C (Eudragit L100-55®), sont réalisés dans les mêmes conditions. Les comprimés de deux formules présentent un format de type D7R5. Ils sont tous terminés à 110 mg.

Tableau 6 :

| Excipients | Formule Eudragit (en %) | Formule témoin (en %) |
|---|---|---|
| Lopéramide HCl (francochim) | 2,00 | 2,00 |
| Eudragit L100-55 (Rhöm, SPCI) | 30,00 | - |
| Kollidon Cl (BASF) | 15,00 | 15,00 |
| Dextrose (Rofèrose G) (Roquette) | 49,7 | 79,7 |
| Sorbitol P100T (Roquette) | 3,00 | 3,00 |
| Stéarate de magnésium (Lambert Rivière) | 0,30 | 0,25 |

<u>Paramètres de technologie pharmaceutique :</u>

Tableau 7

| Formule | Dureté N (Kp) | Temps de désagrégation en seconde * | Friabilité en % |
|---|---|---|---|
| Formule Eudragit | 14,709 ± 3,92 (1,5 ± 0,4) | 9 | 1,17 |
| Formule témoin | 13,82 ± 7,84 (1,41 ± 0,8) | 13 | 0,55 |

*temps de désagrégation testé sur appareil Erweka ZT3.... PH 6,0

L'utilisation de l'Eudragit L100-55 à la proportion de 30 % dans la formule diminue de 30 % le temps de désagrégation du comprimé étudié. De même, il est à noter que la formulation témoin n'est pas satisfaisante d'un point de vue galénique. En effet, lorsqu'on applique une force de compression suffisante pour obtenir un comprimé de dureté égale ou inférieure à 14,70 N (1,5 Kp), le comprimé se clive et colle aux poinçons. Les résultats des essais donnés dans le tableau 7 sont issus de comprimés fabriqués à très faible cadence et triés. Il est clair que cette formulation sans Eudragit ne peut pas faire l'objet d'une fabrication de type industriel.

<u>Exemple comparatif 5 :</u>

Des copolymères d'acide méthacrylique de type A ou B selon l'USP/NF ont été testés pour leur éventuelle propriété d'agent de désagrégation rapide pour les comprimés, dans des conditions comparables à celles observées pour les comprimés comprenant un copolymère d'acide méthacrylique de type C présentés dans les exemples 1 ou 2 ci-dessus.

Les essais A, B, C et D ont été conduits selon le protocole suivant résumé dans le tableau 8. Les comprimés sont de format D9 plat.

Tableau 8 :

| | Essai A (% en masse) | Essai B (% en masse) | Essai C (% en masse) | Essai D (% en masse) |
|---|---|---|---|---|
| Eudragit L100-55 (Rhöm Pharma) | 30,00 | | | |
| Eudragit L100 (Rhöm Pharma) | | | 30,00 | |

Tableau 8 : (suite)

| | Essai A (% en masse) | Essai B (% en masse) | Essai C (% en masse) | Essai D (% en masse) |
|---|---|---|---|---|
| Eudragit S100 (Rhöm Pharma) | | | | 30,00 |
| Kollidon CL (BASF) | 15,00 | 15,00 | 15,00 | 15,00 |
| Dextrose (Roférose G) (Roquette) | 48,75 | 78,75 | 48,75 | 48,75 |
| Sorbitol P100 T (Roquette) | 3,00 | 3,00 | 3,00 | 3,00 |
| Vanille de synthèse (SBI) | 3,00 | 3,00 | 3,00 | 3,00 |
| Huile d'arachide | 0,25 | 0,25 | 0,25 | 0,25 |
| | | | | |
| Masse finale (mg) | 210 | 218 | 218 | 218 |

L'essai A réalisé avec l'Eudragit L100-55, permet d'obtenir des comprimés avec une dureté de 13,72 N (1,4 Kp), un temps de désagrégation de 10 s et une friabilité de 7,4 %.

Dans l'essai B qui correspond à l'essai témoin, les comprimés travaillés de façon à obtenir une dureté d'environ 14,7 N (1,5 Kp) obtiennent des temps de désagrégation se situant entre 20 et 25 s.

Les essais C ont ainsi été réalisés avec de l'Eudragit L100® en tant qu'agent de désagrégation (copolymère d'acide méthacrylique de type A selon l'USP/NF). Des comprimés de 218 mg ont été fabriqués par compression directe. Afin de limiter le temps de désagrégation, les comprimés ont été fabriqués avec la plus faible dureté possible. Etant donnée la mauvaise compressibilité du mélange de poudres la dureté minimale acceptable se situe autour de 29,41 N (31,77 N précisément) (3 Kp (3,24 Kp précisément)). Dans ces conditions, le temps de désagrégation obtenu est de 29,8s, et la mesure de friabilité se révèle impossible : le comprimé se clive. De tels comprimés présentent donc au mieux des caractéristiques de désagrégation moyennes (sans atteindre le désagrégation de type immédiat), et des caractéristiques pharmacotechniques déficientes. Avec un copolymère d'acide méthacrylique de type A, on obtient, de plus, un mélange pulvérulent qui ne présente pas une bonne aptitude à la compression, que cela soit aux cadences de laboratoire ou à celles de l'industrie.

L'utilisation d'un copolymère d'acide méthacrylique de type A selon l'USP/NF en tant qu'agent de désagrégation ne permet donc pas l'obtention de comprimés présentant des caractéristiques de désagrégation et de pharmacotechnie (notamment, dureté, friabilité) comparables à celles observées en utilisant un copolymère d'acide méthacrylique de type C selon l'invention.

Les essais D ont été réalisés de même avec de l'Eudragit S100® en tant qu'agent de désagrégation (copolymère d'acide méthacrylique de type B selon l'USP/NF). Les comprimés sont terminés à 218 mg, ont une dureté de 28,43 N (2,9 Kp) . Un temps moyen de désagrégation de 51,6s a été mesuré. L'utilisation d'un copolymère d'acide méthacrylique de type B USP/NF ne permet pas d'obtenir des comprimés à vitesse de désagrégation appropriée.

La comparaison entre les résultats obtenus avec les copolymères d'acide méthacrylique de type A ou B (ci-dessus illustrés) ou avec les copolymères de méthacrylate d'ammonium de type A ou B, et ceux obtenus avec les copolymères d'acide méthacrylique de type C (illustrés avec l'Eudragit L100-55® dans les exemples 1 à 3 ci-dessus notamment) met en évidence que, de façon surprenante, parmi ces différents polymères de type acrylique, seuls les copolymères d'acide méthacrylique de type C permettent d'obtenir des temps de désagrégation égaux ou inférieurs à 25s en moyenne pour un comprimé ayant une masse maximale se situant autour de 200 mg, tout en conférant aux comprimés de bonnes caractéristiques pharmacotechniques (notamment, dureté, friabilité et stabilité). De manière remarquable, des temps inférieurs à 10s ont pu être enregistrés, tout en observant de très bonnes caractéristiques pharmacotechniques. L'Eudragit L100-55® présente de plus, l'avantage d'être une poudre qui présente une bonne compressibilité, ce qui permet de d'obtenir un comprimé avec une bonne cohésion en travaillant avec des forces de compression faibles. Son utilisation est donc avantageuse au niveau industriel.

## Revendications

1. Procédé de fabrication d'un comprimé, **caractérisé en ce qu'**il comprend la mise en oeuvre d'un copolymère d'acide méthacrylique de type C selon la pharmacopée US National Formulary USP/NF, en tant qu'agent ou co-agent de désagrégation dudit comprimé, et en particulier en tant qu'agent ou co-agent de désagrégation de type immédiat dudit comprimé, **caractérisé en ce qu'**il comprend en outre au moins une étape de mise en forme galénique par simple compression directe, sans nécessiter la mise en oeuvre d'une étape de granulation humide.

2. Procédé selon la revendication 1, **caractérisé en ce qu'**il comprend en outre l'utilisation d'un ou plusieurs agent (s) de désagrégation autre(s) que ledit copolymère, tel que par exemple la crospovidone.

3. Procédé selon l'une quelconque des revendications précédentes, **caractérisé en ce** ladite masse de comprimé se présente sous une forme essentiellement pulvérulente avant mise en forme galénique.

4. Procédé selon l'une quelconque des revendications précédentes, **caractérisée en ce que** ledit copolymère est présent à raison de 5 à 50 % en masse par rapport à la masse totale du comprimé.

5. Comprimé susceptible d'être obtenu à l'aide du procédé selon l'une quelconque des revendications précédentes.

6. Comprimé selon la revendication 5, **caractérisé en ce que** ledit comprimé présente, après mise en forme galénique, une dureté comprise entre 1,96133 N et 98,0665 N environ, une friabilité comprise entre 0 et 25 % environ, un maintien des caractéristiques galéniques au cours du temps.

7. Comprimé selon l'une quelconque des revendications 5 ou 6, **caractérisé en ce que** ledit comprimé ayant une masse se situant autour de 200 mg ou inférieur est capable dans des conditions de désagrégation par voie buccale ou vaginale, de se déliter dans un temps inférieur à 25s environ, préférentiellement dans un temps inférieur ou égal à 20s environ, encore plus préférentiellement inférieur ou égal à 10s environ.

8. Utilisation des procédés selon les revendications 1 à 4 pour la fabrication d'un comprimé adapté, ou destiné, à une application pharmaceutique, vétérinaire et/ou hygiénique.

9. Utilisation selon la revendication 8, **caractérisé en ce que** ledit comprimé est destiné à une administration orale et à une désagrégation dans la cavité buccale.

## Patentansprüche

1. Verfahren zur Herstellung einer Tablette, **dadurch gekennzeichnet, dass** es die Verwendung eines Methacrylsäurecopolymers vom Typ C gemäß der US-Pharmakopöe National Formulary USP/NF als Sprengmittel oder Co-Sprengmittel in der Tablette und insbesondere als sofort wirksames Sprengmittel oder Co-Sprengmittel umfasst, und dass es außerdem mindestens eine Stufe der galenischen Formgebung durch direkte, einfache Komprimierung umfasst und die Stufe einer feuchten Granulierung nicht erforderlich ist.

2. Verfahren nach Anspruch 1, **dadurch gekennzeichnet, dass** es darüber hinaus die Verwendung eines oder mehrerer Sprengmittel wie Crospovidon, die nicht dieses Copolymer sind, umfasst.

3. Verfahren nach einem der vorhergehenden Ansprüche, **dadurch gekennzeichnet, dass** die Tablettenmasse vor der galenischen Formgebung in im Wesentlichen pulvriger Form vorliegt.

4. Verfahren nach einem der vorhergehenden Ansprüche, **dadurch gekennzeichnet, dass** das Copolymer mit einem Anteil von 5 bis 50 Gew.-% der Gesamtmasse der Tablette vorliegt.

5. Tablette, die durch das Verfahren nach einem der vorhergehenden Ansprüche hergestellt werden kann.

6. Tablette nach Anspruch 5, **dadurch gekennzeichnet, dass** sie nach der galenischen Formgebung eine Härte von etwa 1,96133 bis 98,0665 N, eine Bröckligkeit von etwa 0 bis 25 % und eine Beibehaltung der galenischen Eigenschaften im Laufe der Zeit besitzt.

7. Tablette nach Anspruch 5 oder 6, **dadurch gekennzeichnet, dass** sie eine Masse von etwa 200 mg oder darunter besitzt und unter den bei oraler oder vaginaler Aufnahme herrschenden Auflösungsbedingungen innerhalb eines Zeitraums von weniger als etwa 25 s, vorzugsweise weniger als oder gleich etwa 20 s, und besonders bevorzugt weniger als oder gleich etwa 10 s zerfallen kann.

8. Anwendung des Verfahrens nach den Ansprüchen 1 bis 4 auf die Herstellung einer Tablette, die für eine pharmazeutische, veterinärmedizinische und/oder hygienische Verwendung geeignet oder vorgesehen ist.

9. Verwendung nach Anspruch 8, **dadurch gekennzeichnet, dass** die Tablette für eine orale Verabreichung und eine Auflösung in der Mundhöhle vorgesehen ist.

## Claims

1. Process for producing a tablet, **characterized in that** it comprises the use of a methacrylic acid copolymer of type C according to the US Pharmacopea National Formulary USP/NF, as a disintegration agent or co-agent of said tablet, and in particular as an immediate type disintegration agent or co-agent of said tablet, **characterized in that** it also comprises at least one stage of turning into a galenic form by simple direct compression, without requiring the use of a stage of wet granulation.

2. Process according to claim 1, **characterized in that** it further comprises the use of one or more disintegration agent(s) other than said copolymer, such as for example crospovidone.

3. Process according to any one of the preceding claims, **characterized in that** said tablet mass is presented in an essentially pulverulent form before being turned into a galenic form.

4. Process according to any one of the preceding claims, **characterized in that** said copoymer is present in a ratio of 5 to 50% by mass with respect to the total mass of the tablet.

5. Tablet which may be obtained using the process according to any one of the preceding claims.

6. Tablet according to claim 5, **characterized in that**, after being turned into a galenic form, said tablet displays a hardness comprised between approximately 1.96133 N and 98.0665 N, a friability comprised between approximately 0 and 25%, and galenic characteristics which are maintained over time.

7. Tablet according to any one of claims 5 or 6, **characterized in that** said tablet having a mass in the region of 200 mg or less is capable, under oral or vaginal route disintegration conditions, of breaking up in a time of less than approximately 25 seconds, preferentially in a time of less than or equal to approximately 20 seconds, even more preferentially less than or equal to approximately 10 seconds.

8. Use of the processes according to claims 1 to 4 for the preparation of a tablet suitable intended for a pharmaceutical, veterinary and/or sanitary use.

9. Use according to claim 8, **characterized in that** said tablet is intended for oral administration and disintegration in the buccal cavity.